(19) Europäisches Patentamt
European Patent Office
Office européen des brevets

(11) Veröffentlichungsnummer: **0 161 609**
**A2**

(12) # EUROPÄISCHE PATENTANMELDUNG

(21) Anmeldenummer: **85105497.3**

(22) Anmeldetag: **06.05.85**

(51) Int. Cl.⁴: **A 61 K 7/46**, C 08 L 73/00

(30) Priorität: **14.05.84 DE 3417819**

(43) Veröffentlichungstag der Anmeldung: **21.11.85 Patentblatt 85/47**

(84) Benannte Vertragsstaaten: **AT BE CH DE FR GB IT LI NL**

(71) Anmelder: **Henkel Kommanditgesellschaft auf Aktien, Postfach 1100 Henkelstrasse 67, D-4000 Düsseldorf-Holthausen (DE)**

(72) Erfinder: **Schieferstein, Ludwig, Dr., Am Hang 15, D-4030 Ratingen 1 (DE)**
Erfinder: **Zeidler, Ulrich, Dr., Heinrich-Lersch-Strasse 19, D-4000 Düsseldorf 13 (DE)**
Erfinder: **Hensen, Hermann, Dr., Tizianweg 4, D-4010 Hilden (DE)**

(54) **Blockcopolymere Polyglycolether als Lösungsvermittler für Öllösliche Parfümöle.**

(57) Blockcopolymere Polyglycolether aus langkettigen 1,2-Epoxiden und Ethylenoxid der allgemeinen Formel

$$R^1O-\left[C_nH_{2n}O\right]_x \left[C_2H_4O\right]_{x.y}-R^2$$

in der eine der Gruppen $R^1$ oder $R^2$ eine Alkyl- oder Alkoxyalkylgruppe mit 1 bis 22 C-Atomen und die andere Wasserstoff ist, n eine Zahl von 6 bis 22, x eine Zahl von 2 bis 20 und y eine Zahl von 1 bis 100 darstellt, sind geeignet als Lösungsvermittler zur Herstellung klarer, stabiler, wäßriger oder wäßrig-alkoholischer Zubereitungen von öllöslichen Parfümölen. Bevorzugt ist $R^1$ eine Alkylgruppe z.B. eine Methylgruppe und $R^2$ Wasserstoff.

EP 0 161 609 A2

0161609

HENKEL KGaA
ZR-FE/Patente
Dr. JG/Br

Henkelstraße 67
4000 Düsseldorf, den 10. Mai 1984

P a t e n t a n m e l d u n g

D 7007 EP

"Blockcopolymere Polyglycolether als Lösungsvermittler für öllösliche Parfümöle"

Die Erfindung betrifft die Verwendung blockcopolymerer Polyglycolether aus 1,2-Epoxyalkanen und Ethylenoxid als Lösungsvermittler für öllösliche Parfümöle.

Zur Parfümierung klarer, wässriger kosmetischer Zubereitungen wie z.B. Schaum- und Duftbadzubereitungen, Shampoos, flüssiger Seifen, Hautreinigungsmittel, oder wässrig-alkoholischer kosmetischer Zubereitungen wie Gesichtswässer, Rasierwässer, Duftwässer, Haarwässer usw. ist es erforderlich, die Parfümöle, die meist öllösliche ätherische Öle sind, in der Zubereitung klar zu solubilisieren. Die Art und Menge der in den Zubereitungen enthaltenen oberflächenaktiven Stoffe und/oder Lösungsmittel ist oft nicht ausreichend, die öllöslichen Parfümöle in der erforderlichen Menge zu solubilisieren. Aus diesem Grunde ist es erforderlich, lösungsvermittelnde Stoffe, sogenannte Lösungsvermittler oder Solubilisatoren einzusetzen.

Es ist bekannt, als Solubilisatoren für Parfümöle nichtionogene oberflächenaktive Stoffe einzusetzen, z.B. Sorbitanfettsäureester und deren Oxethylate und Oxethylate von hydriertem Rizinusöl. Ein wesentlicher Nachteil vieler Lösungsvermittler besteht darin, daß relativ hohe Zusatzmengen erforderlich sind, um die gewünschte

...

Menge an Parfümöl in Lösung zu bringen. Ein weiterer Nachteil mancher Produkte besteht darin, daß die solubilisierende Wirkung sehr spezifisch ist und sich nur auf einzelne Parfümöle erstreckt. Die Oxethylate des hydrierten Rizinusöls haben darüber hinaus den Nachteil, daß sie als Derivate des Naturstoffes Rizinusöl der schwankenden Verfügbarkeit oder dem damit verbundenen, schwankenden Preis dieses pflanzlichen Öls unterliegen.

Es bestand daher ein dringendes Bedürfnis an Lösungsvermittlern für Parfümöle, die in geringer Einsatzmenge eine gute solubilisierende Wirkung für möglichst zahlreiche öllösliche Parfümöle zur Herstellung stabiler, klarer, wäßriger oder wäßrig-alkoholischer Zubereitungen öllöslicher Parfümöle aufweisen.

Es wurde gefunden, daß blockcopolymere Polyglycolether aus langkettigen 1,2-Epoxyalkanen und Ethylenoxid die gestellten Anforderungen in hohem Maße erfüllen.

Gegenstand der Erfindung ist daher die Verwendung von blockcopolymeren Polyglycolethern aus langkettigen 1.2-Epoxyalkanen und Ethylenoxid der allgemeinen Formel I

$$\text{(I)} \qquad R^1 - O\left[C_n\,H_{2n}O\right]_x \left[C_2H_4O\right]_{x \cdot y} - R^2$$

in der eine der Gruppen $R^1$ oder $R^2$ eine Alkyl- oder Alkoxyalkylgruppe mit 1 bis 22 C-Atomen und die andere Wasserstoff ist, n eine Zahl von 6 bis 22, x eine Zahl von 2 bis 20 und y eine Zahl von 1 bis 100 darstellt, als Lösungsvermittler für öllösliche Parfümöle.

...

Blockcopolymere Polyglycolether der allgemeinen Formel I, in der $R^1$ Wasserstoff und $R^2$ eine Alkylgruppe ist, sind z.B. aus DE-OS 32 07 612 bekannt. Ihre Herstellung erfolgt durch Anlagerung von x Mol langkettiger 1,2-Epoxyalkane der allgemeinen Formel $C_nH_{2n}O$ an Alkylpolyglycolether der allgemeinen Formel

$$R^2\ (OC_2H_4)_{x \cdot y}-OH.$$

Blockcopolymere Polyglycolether der allgemeinen Formel I, in der $R^1$ eine Alkylgruppe oder eine Alkoxyalkylgruppe und $R^2$ Wasserstoff ist, sind als Lösungsvermittler für öllösliche Parfümöle besonders gut geeignet. Sie lassen sich herstellen, indem man x Mol eines linearen 1,2-Epoxyalkans mit n C-Atomen in Gegenwart von 0,1 bis 20 Mol % eines Startalkohols $R^1$-OH und 0,1 bis 5 Mol % eines basischen Katalysators auf eine Temperatur von 100 bis 200 °C so lange erhitzt, bis kein Epoxidsauerstoff mehr nachweisbar ist und auf das entstandene Polymerisat x·y Mol Ethylenoxid in einem Druckgefäß bei einer Temperatur von 100 bis 200 °C einwirken läßt, wobei $R^1$, n, x und y die vorgenannte Bedeutung haben.

Als lineare 1,2-Epoxyalkane können die aus α-Olefinen mit 6 bis 22 C-Atomen durch Epoxidation nach bekannten Verfahren zugänglichen α-Olefinepoxide eingesetzt werden. Es können dabei auch Gemische von 1,2-Epoxyalkanen verschiedener Alkylkettenlängen, wie sie durch Epoxidation von technischen α-Olefinschnitten zugänglich sind, eingesetzt werden.

...

Die Art des Startalkohols ist an sich nicht kritisch. Die Polymerisation läßt sich mit beliebigen einwertigen Alkoholen, die unter den Reaktionsbedingungen zur Öffnung des Epoxidringes geeignet sind, in Gang setzen. Geeignet sind z.B. Methanol, Ethanol, 2-Ethylhexanol, n-Dodecanol, Stearylalkohol, Behenylalkohol. Geeignete Alkohole sind aber auch Methylglycol, Butylglycol und Butyldiglycol. Bevorzugt ist die Verwendung von Alkoholen mit 1 bis 22 C-Atomen, insbesondere von Methanol.

Als basische Katalysatoren können Alkalihydroxide, Alkalialkoholate, Alkali- und Erdalkalisalze organischer Säuren, z.B. Natriumacetat, Calciumacetat verwendet werden. Besonders geeignet ist die Verwendung von Alkalialkoholaten, bevorzugt von Alkoholaten des Startalkohols. Natriummethylat ist der am besten geeignete basische Katalysator.

Zur Polymerisation des 1,2-Epoxyalkans wird das Reaktionsgemisch auf eine Temperatur über 100 °C - ggf. in einem Druckgefäß - erhitzt. Die Reaktion ist beendet, wenn das 1,2-Epoxyalkan umgesetzt ist, d.h. wenn kein freier Epoxidsauerstoff mehr im Reaktionsgemisch nachweisbar ist. Dies ist bei Temperaturen zwischen 100 und 200 °C in 1 bis 10 Stunden, bei einer Temperatur von z.B. 150 °C in ca. drei Stunden erreicht.

Es ist natürlich auch möglich, die Polymerisation des 1,2-Epoxyalkans abzubrechen, bevor das 1,2-Epoxyalkan vollständig umgesetzt ist und nicht umgesetztes 1,2-Epoxyalkan durch Abdestillation unter vermindertem Druck aus dem Reaktionsgemisch abzutrennen. In diesem Falle werden natürlich niedrigere mittlere Polymerisationsgrade erreicht.

...

Auf das entstandene Polymerisat, welches den basischen Katalysator noch enthält, wird dann Ethylenoxid in einem Druckgefäß bei Temperaturen von 100 bis 200 °C, bevorzugt bei 140 bis 180 °C aufgedrückt. Wenn man von einem 1,2-Epoxyalkan-Polymerisat ausgeht, welches vom Katalysator befreit wurde, so ist vor der Ethoxylierung die erneute Zugabe von 2 bis 5 Mol % eines bekannten basischen oder sauren Ethoxylierungskatalysators erforderlich. Die Beendigung der Ethylenoxid-Anlagerung kann an dem damit verbundenen Druckabfall festgestellt werden.

Es ist klar, daß es sich bei den Werten für x und y in Formel I um Mittelwerte handelt, da die Polymerisation bzw. die Anlagerung von Alkylenoxiden stets zu Homologengemischen mit einer statischen Homologenverteilung führt.

In den blockcopolymeren Polyglycolethern der Formel I, in der $R^2$ Wasserstoff ist, läßt sich der mittlere Polymerisationsgrad des 1,2-Epoxyalkans durch die Menge des Startalkohols beeinflußen. Niedrige Zugaben von etwa 0,1 bis 10 Mol % Startalkohol führen zu mittleren Polymerisationsgraden x von über 10, höhere Zugaben von Startalkohol von 10 bis 20 Mol % zu mittleren Polymerisationsgraden x von unter 10.

Polyglycolether der allgemeinen Formel I, in der $R^2$ Wasserstoff ist und bei welchen der mittlere Polymerisationsgrad x zwischen 1 und 2 liegt, stellen ein Gemisch aus den blockcopolymeren Polyglycolethern und Verbindungen dar, wie sie aus DE-PS 23 31 014 bekannt sind. Auch solche Gemische fallen daher in den Bereich der Erfindung.

...

Die nach dem beschriebenen Verfahren erhältlichen, blockcopolymeren Polyglycolether sind weiche bis wachsartig feste Massen, deren Wasserlöslichkeit mit der Menge des angelagerten Ethylenoxids zunimmt. Die Produkte weisen oberflächenaktive Eigenschaften auf. Sie lassen sich bei niedrigen Gehalten an angelagertem Ethylenoxid als Wasser-in-Öl-(W/O)-Emulgatoren, bei höheren Gehalten, z.B. von 10 und mehr Mol Ethylenoxid pro Mol 1,2-Epoxyalkan als Öl-in-Wasser (O/W)-Emulgatoren verwenden. Es wurde festgestellt, daß O/W-Emulsionen, die mit den Blockcopolymeren als Emulgatoren hergestellt wurden, besonders hohe Wärmestabilität aufweisen.

Besonders ausgeprägt ist jedoch das Parfümölsolubilisiervermögen der blockcopolymeren Polyglycolether.

Die blockcopolymeren Polyglycolether ermöglichen die Herstellung klarer, stabiler, wässriger und wässrig-alkoholischer Zubereitungen von öllöslichen ätherischen Ölen und Parfümölen bereits mit verhältnis niedrigen Einsatzmengen der Lösungsvermittler. Unter wässrigalkoholischen Zubereitungen werden Zubereitungen in Mischungen aus Wasser und niederen Alkoholen wie Ethanol und Isopropanol verstanden. Das Mischungsverhältnis von Wasser zu niederem Alkohol kann dabei beliebig, bevorzugt im Bereich von 90 : 10 bis 10 : 90 liegen. Für die Herstellung klarer, stabiler wässriger und wässrig-alkoholischer Zubereitungen ist es erforderlich, Mengen von 0,1 bis 10 Gewichtsprozent der Parfümöle klar zu solubilisieren, Mengen von 0,5 bis 2,0 Gewichtsprozent klar solubilisiertes Parfümöl sind aber für die meisten Zwecke ausreichend.

...

Für die klare Solubilisierung zahlreicher ätherischer Öle sind bereits Mengen von 0,3 bis 5,0 Gewichtsteilen der Polyglycolether der allgemeinen Formel I, bezogen auf 1 Gewichtsteil Parfümöl ausreichend. Besonders ausgeprägt ist die lösungsvermittelnde Wirkung für Parfümöle bei solchen Polyglycolethern der allgemeinen Formel I, in der n eine Zahl von 8 bis 16, x eine Zahl von 2 bis 10 und y eine Zahl von 10 bis 50 darstellt.

Die folgenden Beispiele sollen den Gegenstand der Erfindung näher erläutern ohne ihn hierauf zu beschränken.

...

Beispiele

1. Herstellung der blockcopolymeren Polyglycolether (in welchen $R^2$ Wasserstoff ist)

Verfahrensbeschreibung am Beispiel des Produktes 1.4 (Tabelle)

In einem Stahlautoklaven wurden 184 g (1,0 Mol) 1-Epoxydodecan und 5,6 g einer 30 gewichtsprozentigen methanolischen Lösung von Natriummethylat (0,03 Mol Na-Methylat) unter Rühren drei Stunden lang auf 150 °C erhitzt. Nach dieser Zeit konnte in einer Probe des Reaktionsgemisches kein Epoxidsauerstoff mehr nachgewiesen werden. Danach wurden bei 150 °C portionsweise nach und nach 880 g (20 Mol) Ethylenoxid unter Druck eingeleitet. Nach 5 Stunden konnte am Druckabfall festgestellt werden, daß das Ethylenoxid umgesetzt war.

Nach Abkühlung und Entspannung wurde ein wachsartiges, wasserlösliches Produkt mit einer Hydroxylzahl von 12 erhalten.

Aus dem Molekulargewicht des α-Epoxids (EPO), des Ethylenoxids (EO), des Polyglycolethers (MG) gemäß Hydroxylzahl und der pro Mol α-Epoxid angelagerten Molmenge an Ethylenoxid y kann der mittlere Polymerisationsgrad des α-Epoxids x wie folgt abgeschätzt werden:

$$x = \frac{MG}{EPO + y\ EO}$$

(Der Gewichtsanteil des Startalkohols ($CH_3OH$) bleibt unberücksichtigt)

...

1.1 - 1.9 (Tabelle)

Nach den unter 1.1 beschriebenen Herstellverfahren wurden die in der nachfolgenden Tabelle aufgeführten blockcopolymeren Polyglycolether hergestellt. In allen Fällen wurde Methanol als Startalkohol verwendet. Es wurden unterschiedliche Mengen an Startalkohol eingesetzt.

...

| Nr. | 1,2-Epoxy-alkan (n) | angelagertes EO (Mol pro Mol Epoxid) (y) | OHZ | x | Beschaffenheit | 10 Gew.% in Wasser |
|---|---|---|---|---|---|---|
| 1.1 | 8 | 10 | 30 | 3,28 | wachsartig fest | klar gelöst |
| 1.2 | 12 | 5 | 34,5 | 4,02 | weiche Masse | trübe Lösung |
| 1.3 | 12 | 10 | 24,9 | 3,60 | wachsartig fest | trübe Lösung |
| 1.4 | 12 | 20 | 12,0 | 4,38 | wachsartig fest | klar gelöst |
| 1.5 | 12 | 50 | 14,0 | 1,68 | wachsartig fest | klar gelöst |
| 1.6 | 16 | 1 | 29,5 | 6,68 | weiche Masse | trüb mit Bodenkörper |
| 1.7 | 16 | 5 | 19,9 | 6,12 | weiche Masse | trüb mit Bodenkörper |
| 1.8 | 16 | 10 | 11,9 | 6,92 | wachsartig fest | trüb mit Bodenkörper |
| 1.9 | 16 | 50 | 9,4 | 2,44 | wachsartig fest | klar gelöst |

2. Parfümölsolubilisiervermögen

Das Parfümölsolubilisierungsvermögen der blockcopolymeren Polyglycolether wurde wie folgt geprüft:

Es wurden Mischungen aus 1 g eines bestimmten, fettlöslichen Parfümöls und steigenden Mengen, (z.B. 1 g, 2 g, 3 g, 4 g usw.) des Lösungsvermittlers (Polyglycolether) hergestellt. Jede dieser Mischungen wurde dann mit Wasser bzw. mit einem Wasser-Alkohol-Lösungsmittelgemisch auf 100 g aufgefüllt. Auf diese Weise wurden Lösungen von 1 Gewichtsprozent des Parfümöls erhalten. Ab einem bestimmten, zur Solubilisierung ausreichenden Gehalt an Lösungsvermittler waren die erhaltenen Lösungen bei 20 °C klar. Diese, zur klaren Solubilisierung von 1 Gewichtsprozent des jeweiligen Parfümöls erforderliche Menge an Solubilisator ist in der Tabelle II für ein Lösungsmittelgemisch aus 75 Gewichtsprozent Wasser und 25 Gewichtsprozent Ethanol für einige der in Tabelle I aufgeführten erfindungsgemäßen Polyglycolether angegeben. Zum Vergleich sind die erhaltenen Werte für einen handelsüblichen Parfümölsulibilisator - das Anlagerungsprodukt von 60 Mol Ethylenoxid an hydriertes Rizinusöl ("RH 60") - aufgeführt.

Es wurden die folgenden Parfümöle eingesetzt:

A Pineöl
B Nelkenblätteröl
C Rosmarinöl
D Lavandinöl
E Pfefferminzöl
F Bergamotteöl
G Cedernholzöl
H Patchouliöl
J Zitronenöl
K Organgenöl

...

T a b e l l e II

| Lösungsvermittler | Lösungsmittel Wasser : Ethanol (Gew.%) | Zur Solubilisierung von 1 Gew.% Parfümöl (A-J) erforderliche Menge (Gew.%) Lösungsvermittler | | | | | | | | | |
|---|---|---|---|---|---|---|---|---|---|---|---|
| | | A | B | C | D | E | F | G | H | J | K |
| 1.1 | 75 : 25 | 1 | 2 | 2 | 2 | 2 | 2 | 4 | 4 | 1 | 3 |
| 1.3 | 75 : 25 | 1 | 2 | 2 | 2 | 2 | 4 | 7 | 7 | 3 | 5 |
| 1.4 | 75 : 25 | 1 | 2 | 2 | 2 | 2 | 3 | 5 | > 7+) | 2 | 2 |
| 1.5 | 75 : 25 | 3 | 3 | 2 | > 7 | 5 | 3 | > 7 | > 7 | 4 | 3 |
| 1.9 | 75 : 25 | 3 | 3 | 1 | 7 | 4 | 3 | > 7 | 7 | 2 | 3 |
| "RH 60" | 75 : 25 | 1 | 3 | 2 | 2 | 1 | 2 | 5 | 2 | 2 | 2 |
| 1.4 | 35 : 65 | 1 | 1 | 1 | 1 | 1 | 1 | 1 | 1 | 1 | 1 |
| 1.5 | 35 : 65 | 1 | 1 | 1 | 1 | 1 | 2 | 2 | 4 | 2 | 2 |
| 1.9 | 35 : 65 | 1 | 1 | 1 | 1 | 1 | 2 | 2 | 2 | 2 | 2 |
| "RH 60" | 35 : 65 | 1 | 1 | 1 | 1 | 1 | 1 | 2 | 2 | 1 | 1 |

+) > 7 bedeutet: die mit 7 Gew.% Lösungsvermittler erhaltene Lösung war (bei 20 °C) noch nicht ganz klar.

## Patentansprüche

1. Verwendung von blockcopolymeren Polyglycolethern aus langkettigen 1,2-Epoxyalkanen und Ethylenoxid der allgmeinen Formel I

$$\text{(I)} \qquad R^1O - \left[C_nH_{2n}O\right]_x \left[C_2H_4O\right]_{x \cdot y} - R^2$$

in der eine der Gruppen $R^1$ oder $R^2$ eine Alkyl- oder Alkoxyalkylgruppe mit 1 bis 22 C-Atomen und die andere Wasserstoff ist, n eine Zahl von 6 bis 22, x eine Zahl von 2 bis 20 und y eine Zahl von 1 bis 100 darstellt, als Lösungsvermittler für öllösliche Parfümöle.

2. Verwendung nach Anspruch 1, dadurch gekennzeichnet, daß $R^1$ eine Alkyl- oder Alkoxyalkylgruppe und $R^2$ Wasserstoff ist.

3. Verwendung nach Anspruch 2, dadurch gekennzeichnet, daß $R^1$ eine Methylgruppe ist.

4. Klare, stabile, wässrige oder wässrig-alkoholische Zubereitungen von öllöslichen Parfümölen, gekennzeichnet durch einen Gehalt von blockcopolymeren Polyglycolethern der allgemeinen Formel I nach Anspruch 1 in einer Menge von 0,3 bis 5 Gewichtsteilen, bezogen auf 1 Gewichtsteil Parfümöl.